# EUROPEAN PATENT SPECIFICATION

(11) **EP 3 738 561 B1**
(45) Date of publication and mention of the grant of the patent: **30.11.2022**
(21) Application number: 18902853.3
(22) Date of filing: 30.01.2018
(51) Int. Cl.: A61F 13/532, A61F 13/49

(54) **ABSORBENT ARTICLE**
ABSORBIERENDER ARTIKEL
ARTICLE ABSORBANT

(30) Priority: 29.01.2018 JP 2018012882
(43) Date of publication of application: 18.11.2020
(73) Proprietor: Unicharm Corporation, Shikokuchuo-shi, Ehime 799-0111 (JP)
(72) Inventor: SAKAGUCHI, Satoru, Kanonji-shi, Kagawa 769-1602 (JP); YAMANAKA, Yasuhiro, Kanonji-shi, Kagawa 769-1602 (JP); TAKAHASHI, Maika, Kanonji-shi, Kagawa 769-1602 (JP)
(74) Representative: Dolleymores
(86) International application number: PCT/JP2018/002947
(87) International publication number: WO 2019/146126

(56) References cited:
- EP-A2- 1 384 459
- WO-A1-2011/105109
- WO-A1-2013/005397
- JP-A- 2004 049 765
- JP-A- 2016 120 017
- JP-A- 2017 217 160

## Description

### [TECHNICAL FIELD]

The present invention relates to a tape type absorbent article such as a disposable diaper.

### [BACKGROUND ART]

An absorbent article described in Patent Literature 1 is a tape type absorbent article including an absorbent core and side flaps extending outward in the width direction from the absorbent core. To put on the absorbent article thus configured, the buttocks of the wearer are first put on the rear waistline region of the absorbent article with the absorbent article in a deployed state. Then, the absorbent article is passed between the legs of the wearer and is pulled up to cover the abdomen of the wearer.

### [CITATION LIST]

### [PATENT LITERATURE]

Patent Literature 1: JP 2017-63848 A

JP 2004049765

A relates to a diaper having a crotch leakage prevention function by the combination of three-dimensional guards and crotch elastic members.

EP 1384459 A2 discloses a disposable diaper having a configuration such that a plurality of leg portion elastic members are arranged around the respective leg openings of the disposable diaper so that the respective leg portion elastic members are not connected or cross each other in a crotch portion.

WO 2011/105109 A1 and WO 2013/005397 A1 both disclose pants-type diapers with a central slit, a pair of side slits and a central elastic member.

### [SUMMARY OF INVENTION]

The absorbent article thus configured has the side flaps extending outward in the width direction from the absorbent core. The side flaps easily hit legs of the wearer at the time of wearing. When the side flaps hit the legs at the time of wearing, it may sometimes be difficult to pull up the absorbent article to the abdomen. To ensure ease of pulling up at the time of wearing, the length in the width direction of the absorbent core may be shortened. With a shorter length of the absorbent core in the width direction, however, may not achieve a sufficient absorption area for the body fluid.

Therefore, it is desired to provide an absorbent article that can be pulled up easily at the time of wearing and can reliably provide an absorption area for body fluid.

The present invention provides the absorbent article of independent claim 1. The dependent claims specify preferred but optional features.

### [BRIEF DESCRIPTION OF DRAWINGS]

Fig. 1 is a schematic plan view of an absorbent article according to an embodiment.
Fig. 2 is a schematic cross-sectional view taken along line A-A of the absorbent article illustrated in Fig. 1.
Fig. 3 is a schematic cross-sectional view taken along line B-B of the absorbent article illustrated in Fig. 1.
Fig. 4 schematically illustrates a wearing state of the absorbent article in the cross-sectional view taken along line A-A.
Fig. 5 schematically illustrates a wearing state of the absorbent article in the cross-sectional view taken along line B-B.
Fig. 6 schematically illustrates a wearing state of the absorbent article according to the embodiment.

### [DESCRIPTION OF EMBODIMENTS]

### (1) Outline of Embodiment

According to the present specification and the accompanying drawings, at least the following matters will be disclosed.

An absorbent article according to one embodiment includes a front-back direction, a width direction orthogonal to the front-back direction, an absorbent core including an absorbent material, a side flap extending outward in the width direction from the absorbent core, and a fastening tape provided on the side flap, in which the absorbent article includes a front region, a crotch region, and a rear region which are obtained by equally dividing a total length of the absorbent core in the front-back direction into three parts, a length of the side flap in the width direction in the crotch region is at least 30 mm, the crotch region includes a central region and side regions located on both outer sides of the central region in the width direction, low basis weight portions each having a basis weight of the absorbent material lower than a basis weight of the surroundings thereof are provided on both outer edges of the central region in the front-back direction, and the central region has a contraction force in the front-back direction higher than a contraction force of the side regions in the front-back direction.

According to the present embodiment, when the absorbent article is pulled up to bring the crotch region closer to the wearer at the time of wearing, the crotch region is deformed in a convex shape in which the central region protrudes more toward the body than the side regions. As the absorbent core in the crotch region deforms in the convex shape, the length in the width direction of the entire absorbent core at the time of wearing is shortened. The side flaps do not easily hit the legs of the wearer at the time of wearing, and the absorbent article is easily pulled up. Therefore, wearability of the absorbent article (ease of wearing) is improved. Since the ease of pulling up the absorbent article is achieved by the deformation of the absorbent core in the convex shape in the crotch region, it is possible to reliably provide the length in the width direction of the entire crotch region and the absorption area for body fluid.

According to the embodiment, the central region includes a central elastic member that stretches in the front-back direction.

According to the present embodiment, the central elastic member stretches, so that the absorbent core in the crotch region can deform more easily in the convex shape in which the central region protrudes more toward the wearer than the side regions. Accordingly, the side flaps hardly hit the legs of the wearer at the time of wearing, and the absorbent article is more easily pulled up.

According to a preferred embodiment, the central elastic member may be disposed outside the center of a region between the center of the central region and the outer edge of the central region in the width direction.

According to the present embodiment, the central elastic member is located outside the center of a region between the center of the central region and the outer edge of the central region in the width direction, so that the contraction force by the central elastic member easily acts on the low basis weight portion. The low basis weight portion is easily pulled up to the body side by both the difference in rigidity due to the difference in basis weight and the contraction force of the central elastic member. Accordingly, the absorbent core in the crotch region is deformed in the convex shape, and the ease of pulling up at the time of wearing is easily achieved.

According to a preferred embodiment, the central elastic member may be disposed overlapping the low basis weight portion.

According to the present embodiment, the contraction force by the central elastic member easily acts on the low basis weight portion. The low basis weight portion is easily pulled up to the body side by both the difference in rigidity due to the difference in basis weight and the contraction force of the central elastic member, the absorbent core in the crotch region deforms into the convex shape, and the ease of pulling up at the time of wearing is easily achieved.

According to a preferred embodiment, the central elastic member may be disposed in a pair at a position not overlapping and apart from a widthwise center of the central region in the width direction.

According to the present embodiment, a region not overlapping the central elastic member is provided between the low basis weight portions. The region not overlapping the central elastic member is more easily disposed away from the body at the time of wearing than the region where the central elastic member is disposed and the region where the low basis weight portions is provided. By forming a space between the low basis weight portions and the body, it is possible to reliably provide a space for keeping the body fluid and prevents leakage of the body fluid.

According to a preferred embodiment, the central elastic member may be disposed on the non-skin contact surface side of the absorbent core.

According to the present embodiment, the central elastic member is disposed on the non-skin contact surface side of the absorbent core, so that the absorbent core is easily pushed up toward the wearer by the central elastic member. Accordingly, the absorbent core in the crotch region more easily deforms in the convex shape in which the absorbent core is disposed closer to the body by the central elastic member and the central region protrudes more toward the wearer than the side regions. An effect of easily pulling up at the time of wearing is easily obtained, while reliably providing the absorption area of body fluid.

According to a preferred embodiment, the central elastic member may be disposed in a region not overlapping the absorbent core in the side regions.

According to the present embodiment, the central elastic member does not overlap the absorbent core in the side regions, the central region is more easily pulled toward the body than the side regions. The central region is easily deformed in the convex shape protruding toward the body. An effect of easily pulling up at the time of wearing is easily obtained, while reliably providing the absorption area of body fluid.

According to a preferred embodiment, the central elastic member is disposed across a folding line formed by folding the absorbent article in two in the front-back direction, and the center in the front-back direction of the central elastic member may be disposed behind the center of the absorbent article in the front-back direction.

According to the present embodiment, the central elastic member is disposed across the folding line, so that a crease can be brought closer to the body. Since the central elastic member is disposed on the rear side of the absorbent article, the rear side of the absorbent article in the front-back direction can be more contracted. The region where the body is placed at the time of wearing is disposed closer to the region that is contracted by the central elastic member, so that the region extending from the body at the time of wearing can easily fit the body at the time of wearing. The absorbent core is disposed to fit the body to prevent the absorbent core from getting caught by the legs at the time of wearing, thus facilitating the pull-up. By disposing the absorbent core to fit the body, the absorption area of the body fluid is reliably provided.

According to a preferred embodiment, the rear edge of the central elastic member may be located in front of the region ranging up to 1/3 of the total length of the absorbent article in the front-back direction from the rear edge of the absorbent article.

According to the present embodiment, the region ranging up to 1/3 of the total length of the absorbent article in the front-back direction from the rear edge of the absorbent article often functions as a region on which the body of the wearer is placed at the time of wearing. Since the central elastic member is not provided in the region where the body is placed at the time of wearing, the body is placed on a smooth absorbent core to allow the absorbent core to fit the body. By disposing the absorbent core to fit the body, it is possible to reliably provide the absorption area of the body fluid.

According to a preferred embodiment, the rear edge of the central elastic member may be located in front of the rear edge of the low basis weight portion.

According to the present embodiment, in the area behind the low basis weight portion, the contraction force by the central elastic member hardly acts and the smooth state can be maintained easily. In the region behind the low basis weight portion, the rear end portion of the absorbent article on which the body is placed at the time of wearing can easily fit the body in the smooth state. By disposing the absorbent core to fit the body, it is possible to reliably provide the absorption area of the body fluid.

According to a preferred embodiment, the distance between the low basis weight portions may be not more than 1/2 of the maximum length in the width direction of the absorbent core.

According to the present embodiment, the low basis weight portions are disposed to approach to the body at the time of wearing. Since the distance between the low basis weight portions is not more than 1/2 of the maximum length of the absorbent core, the low basis weight portions are easily disposed between the legs of the wearer and the convex shape of the absorbent core is easily maintained. Accordingly, the side flaps hardly hit the legs of the wearer at the time of wearing, and the absorbent article is more easily pulled up.

According to a preferred embodiment, the distance between the low basis weight portions may be not more than 1/2 of the minimum length in the width direction of the absorbent core in the crotch region.

According to the present embodiment, the distance between the low basis weight portions is not more than 1/2 of the minimum length of the absorbent core in the crotch region, so that the absorbent core is easily disposed between the legs of the wearer and the convex shape of the absorbent core is easily maintained. Accordingly, the side flaps hardly hit the legs of the wearer at the time of wearing, and the absorbent article is more easily pulled up.

According to a preferred embodiment, bending stiffness of the central region in the width direction may be higher than the bending stiffness of the side regions in the width direction.

According to the present embodiment, the bending stiffness of the central region in the width direction is higher than the bending stiffness in the width direction of the side region, the center region is easily maintained in a smooth state even when the center region has a high contraction force. Accordingly, the absorption area of body fluid is reliably provided more easily. Further, the absorbent core is easily disposed to fit the body, so that the absorbent core is prevented from being caught by the legs at the time of wearing and is easily pulled up.

According to a preferred embodiment, the central region may be formed with a compressed portion formed by at least squeezing the absorbent core.

According to the present embodiment, the compressed portion increases the bending stiffness of the central region, and the central region is maintained in a smooth state and is disposed to fit the body. Accordingly, the absorption area of body fluid is reliably provided more easily. Further, the absorbent core is easily disposed to fit the body, so that the absorbent core is prevented from being caught by the legs at the time of wearing and is easily pulled up.

According to a preferred embodiment, the compressed portion may be disposed between the low basis weight portions.

According to the present embodiment, the region between the low basis weight portions is maintained in a smooth state and is disposed to fit the body. Accordingly, the absorption area of body fluid is reliably provided more easily. Further, the absorbent core is easily disposed to fit the body, so that the absorbent core is prevented from being caught by the legs at the time of wearing and is easily pulled up.

According to a preferred embodiment, the central region may include the central elastic member that stretches in the front-back direction, and the central elastic member may be disposed on the outside of the compressed portion in the width direction.

According to the present embodiment, the region where the central elastic member is disposed is pulled up toward the body by the contraction of the central elastic member, and the region between the central elastic members can easily be disposed to fit the body by the compressed portion. The space between the central elastic members easily deforms into the convex shape to fit the body. Therefore, while reliably providing the absorption area of the body fluid, it is easier to obtain the effect of being easily pulled up at the time of wearing.

### (2) Overall Structure of Absorbent Article

An absorbent article according to an embodiment will be described below by referring to the accompanying drawings. In the drawings, the same or similar parts are indicated by the same or similar reference signs. The drawings are illustrated schematically, and dimensional ratio and other variables differ from those of actual measurements. The actual measurements or the like, therefore, should be determined by referring to the following description. The drawings may include different relationships or ratios of measurements.

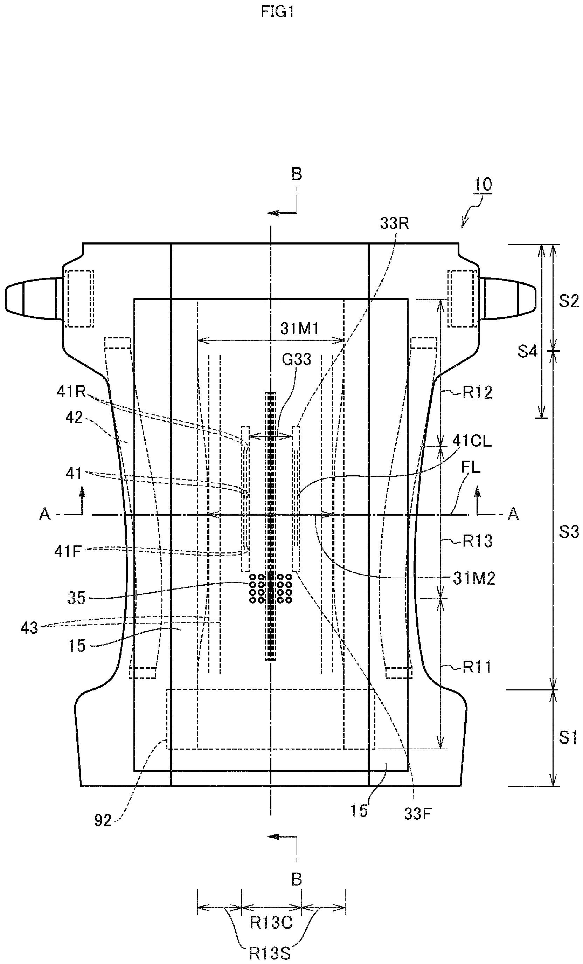

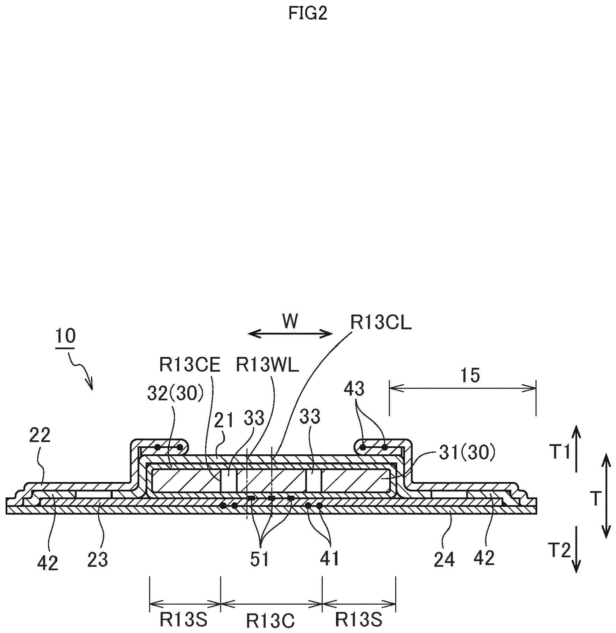

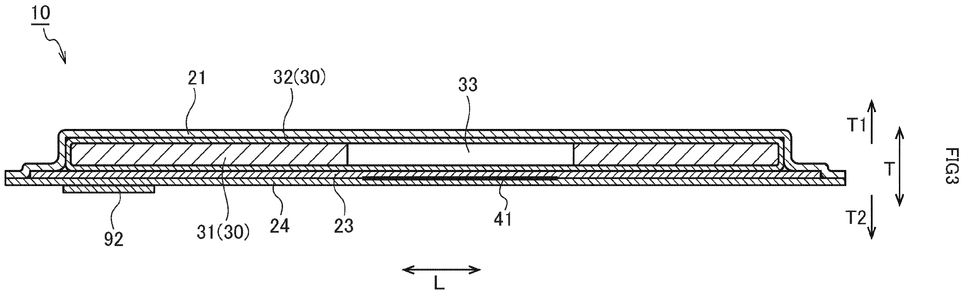

The absorbent article is a tape type disposable diaper. Fig. 1 is a plan view of the absorbent article 10 according to the embodiment. The schematic plan view of Fig. 1 illustrates the disposable diaper in an extended state. Fig. 2 is a schematic cross-sectional view taken along line A-A of the absorbent article illustrated in Fig. 1. Fig. 3 is a schematic cross-sectional view taken along the line B-B of the absorbent article illustrated in Fig. 1. As used herein, the extended state in the present invention refers to a state in which the absorbent article 10 is extended till no wrinkles are formed. Further, a natural state, as used herein, in the present invention refers to a state in which, in the case of an absorbent article 10 put in the package, the absorbent article 10 is taken out from the package, and left at 20°C ± 2°C and a relative humidity of 60% ± 5% RH in the atmosphere for 24 hours.

The absorbent article 10 includes a front-back direction L and a width direction W which are orthogonal to each other. The front-back direction L is defined by the directions extending to the front side of the body and the rear side of the body. In other words, the front-back direction L is a direction extending forward and backward in the deployed state of the absorbent article 10. The absorbent article 10 also includes a thickness direction T perpendicular to both the front-back direction L and the width direction W. The thickness direction T extends to a skin contact surface side T1 facing the wearer and the non-skin contact surface side T2 facing away from the skin contact surface side.

The absorbent article 10 includes a front waistline region S1, a rear waistline region S2, and an intermediate region S3. The front waistline region S1 is a region facing the front waistline (abdomen) of the wearer. The rear waistline region S2 is a region facing the rear waistline (back) of the wearer and includes a region on which the body (buttocks) is placed at the time of wearing. The intermediate region S3 is located in the crotch of the wearer and is a region disposed between the front waistline region S1 and the rear waistline region S2. The intermediate region S3 is a region including a leg surrounding opening disposed around the legs of the wearer. The leg surrounding opening is a portion recessed inward in the width direction from the outer edge of the absorbent article.

The absorbent article 10 includes an absorbent core 31 that includes an absorbent material. The absorbent core 31 includes an absorbent material such as ground pulp or superabsorbent polymer (SAP), or mixtures thereof. The absorbent core 31 may be covered by a core wrap 32. The absorbent core 31 and the core wrap 32 form an absorber. The core wrap 32 is made of a tissue and may be disposed on the skin contact surface side T1 of the absorbent core 31 and the non-skin contact surface side of the absorbent core.

As illustrated in Figs. 1 and 3, the absorbent article 10 includes a front region R11, a crotch region R13, and a rear region R12 obtained by equally dividing the total length of the absorbent core 31 into three parts in the front-back direction. The crotch region R13 is disposed behind the front region R11 and a rear region R12 is disposed behind the crotch region R13. The front region R11, the crotch region R13, and the rear region R12 have the same length in the front-back direction L.

The crotch region R13 includes a central region R13C and side regions R13S located on both outer sides of the central region R13C in the width direction W. The central region R13C may be disposed across the center of the absorbent core 31 in the width direction W. The side regions R13S may be disposed on both sides of the central region R13C in the width direction. A low basis weight portion 33 may be provided on both outer edges of the central region R13C. The low basis weight portion 33 may be provided at least on the outer edge of the central region R13C, and may extend inward in the width direction W from the outer edge of the central region R13C. The outer edge of the low basis weight portion 33 defines the outer edge of the central region R13C. In other words, the outer edge of the low basis weight portion 33 defines the border between the side regions R13S and the central region R13C. As used herein, the outer portion in the present invention refers to a portion that occupies a certain range in the width direction W including the outer edge in the width direction W, and the outer edge refers to the outer edge in the width direction W. In addition, the inner portion refers to a portion that occupies a certain range in the width direction W including the inner edge in the width direction W, and the inner edge refers to the inner edge in the width direction W.

The low basis weight portion 33 is provided along the front-back direction L. The low basis weight portion 33 may extend in the front-back direction L, may be parallel to the front-back direction L, or may be inclined at an angle of less than 90 degrees with respect to the front-back direction L. The low basis weight portion 33 is a portion having a basis weight of the absorbent material lower than a basis weight of the surrounding absorbent core 31, is thinner than the surrounding absorbent core 31, and has low rigidity. Therefore, the low basis weight portion 33 tends to become a base point of deformation in the wearing state. The base point of deformation extending in the front-back direction is easily formed in the absorbent core 31 in the attached state. The low basis weight portion 33 may be a portion in which the basis weight of the absorbent material is substantially zero, or may be a portion including the absorbent material having a smaller basis weight than a basis weight of the surroundings thereof. The low basis weight portion 33 may be a slit having a fixed length in the width direction W, or a slit in which the length in the width direction W is substantially zero.

The low basis weight portion 33 may be provided at least in the crotch region R13 and may extend from the crotch region R13 to the rear region R12, or may extend from the crotch region R13 to the front region R11. A front edge 33F of the low basis weight portion 33 may be located in the crotch region R13, and a rear edge 33R of the low basis weight portion 33 may be located in the rear region R12. The length of each low basis weight portion 33 in the width direction W may be from 2 mm to 10 mm, and the length of the low basis weight portion 33 in the front-back direction L may be from 30 mm to 180 mm.

A compressed portion 35 may be formed in the absorbent core 31. The compressed portion 35 needs to be formed by squeezing at least the absorbent core 31 in a thickness direction T, or may be formed by pressing the absorbent core 31 with other members such as the core wrap 32, and the absorbent core 31. The compressed portion 35 may be provided at least in the central region R13C. The compressed portion 35 of the present embodiment is dot-like, and a plurality of compressed portions 35 are provided at intervals in the front-back direction L and the width direction W. The compressed portion 35 is provided in front of the low basis weight portions 33 and in a region between the low basis weight portions 33 in the width direction W.

As illustrated in Figs. 2 and 3, a topsheet 21 and side sheets 22 may be provided on the skin contact surface side T1 of the absorber 30 including the absorbent core 31. The topsheet 21 may be disposed across the center of the absorber 30 in the width direction W. The side sheets 22 may be disposed to cover the outer portion of the topsheet 21. The topsheet 21 and the side sheets 22 may be made of a liquid-permeable sheet such as a nonwoven fabric or a perforated plastic film. The inside portion of each side sheet 22 may be folded back and overlapped. A leakage preventing elastic member 43 that stretches in the front-back direction L may be provided between the overlapped portion of the side sheets 22. The leakage preventing elastic member 43 may be made of a thread rubber which stretches in the front-back direction L. The side sheet 22 and the leakage preventing elastic member 43 form leakage preventing gathers uprising on the skin contact surface side T1 during wearing.

On the non-skin contact surface side T2 of the absorber 30, a backsheet 23 and an exterior sheet 24 may be provided. The exterior sheet 24 may be provided on the non-skin contact surface side T2 of the backsheet 23. The length in the width direction W of the backsheet 23 is shorter than the length in the width direction W of the exterior sheet 24, and the length in the front-back direction L of the backsheet 23 is shorter than the length in the front-back direction L of the exterior sheet 24. The backsheet 23 may be made of a liquid-impermeable film. The exterior sheet 24 may be formed of a liquid-permeable nonwoven fabric.

Indicators 51 may be disposed between the backsheet 23 and the absorber 30. The indicators 51 extend in the front-back direction L and is disposed at intervals in the width direction W. The indicators 51 are disposed between the low basis weight portions 33 in the width direction W. Each indicator 51 changes its color as the absorber absorbs the body fluid, so that the absorption state of the body fluid can be grasped from the non-skin contact surface side T2 of the absorbent article 10.

A leg elastic member 42 extending in the front-back direction L may be provided between the side sheets 22 and the backsheet 23 and between the side sheets 22 and the exterior sheet 24. The leg elastic member 42 may be made of a band-like stretchable sheet that stretches in the front-back direction L. By the contraction of the leg elastic member 42, the absorbent article 10 fits around the legs during wearing.

The absorbent article 10 includes side flaps 15 extending outward in the width direction W from the absorbent core 31. Each side flap 15 is a portion extending outward in the width direction W from the absorbent core 31. The side flaps 15 are provided between the outer edge of the absorbent core 31 and the outer edge of the absorbent article 10. As illustrated in Fig. 2, the side flaps 15 may be formed by the topsheet 21, the side sheets 22, the leg elastic member 42, the backsheet 23, and the exterior sheet 24. The length of each side flap 15 in the crotch region R13 in the width direction may be from 30 mm to 100 mm. Since the side flaps 15 extend at least 30 mm in the crotch region than the absorbent core 31, the side flaps 15 easily hit the legs of the wearer at the time of wearing. When the side flaps 15 hit the legs at the time of wearing, pulling up the absorbent article to the abdomen may be difficult. The length in the width direction of the side flaps may be at least 30 mm in at least a part of the crotch region. In other words, in the configuration in which the length of the side flaps change in the crotch region, the side flaps may have a portion having a length of at least 30 mm and a portion having a length of less than 30 mm. Since the side flaps 15 having the length of at least 30 mm in the width direction are provided at least partially, the side flaps 15 easily come to hit the legs at the time of wearing. The absorbent article 10 of the present embodiment is configured such that the side flaps 15 do not easily hit the legs at the time of wearing. The configuration in which the side flaps 15 do not easily hit the legs will be described in detail later in describing the mounting state.

A fastening tape 91 is attached to each of the side flaps 15. The fastening tape 91 extends in the width direction W in the rear waistline region S2 and is fastened to a target portion 92 to hold the absorbent article 10 on the body of the wearer. The target portion 92 is configured so that the fastening tape 91 is put in the front waistline region S1.

In the crotch region R13, the contraction force in the front-back direction L of the central region R13C is higher than the contraction force in the front-back direction L of the side regions R13S. The central region R13C tends to shrink more in the front-back direction L than the side regions R13S. Since the central elastic member 41 is disposed in a region overlapping the central region R13C, the contraction force in the front-back direction L of the central region R13C may be higher than the contraction force in the front-back direction L of the side regions R13S. The central region R13C is more likely to contract than the side regions R13S due to the contraction of the central elastic member 41. The central elastic member 41 is provided so that the absorbent core 31 in the crotch region R13 is deformed in a convex shape in which the central region R13C protrudes toward the wearer from the side regions R13S. Note that the contraction force, as used herein, refers to a contraction force in the product state of the absorbent article 10. According to the effective length and the contraction in the natural state of the elastic member (the central elastic member 41 of the present embodiment) which stretches in the front-back direction L, it is possible to compare the contraction forces of the region R13S and the central region R13C. The region having a large contraction mode in the natural state is a region having a high contraction force.

Note that the central elastic member 41 refers to a portion in which an elastic member such as thread rubber is disposed in a stretchable manner in the front-back direction L, and does not include a portion in which the elastic member is disposed in a non-stretchable manner in the front-back direction L. In other words, the central elastic member 41 is an effective length portion of the elastic member that forms the central elastic member 41. As used herein, in the present invention, the overlapping state refers to a state in which one member overlaps the other member in a plan view, and the other member is disposed on the skin contact surface side or non-skin contact surface side of one member. The overlapping state includes not only a state in which one member and the other member are in contact with each other, but also a structure in which another member is interposed between one member and the other member.

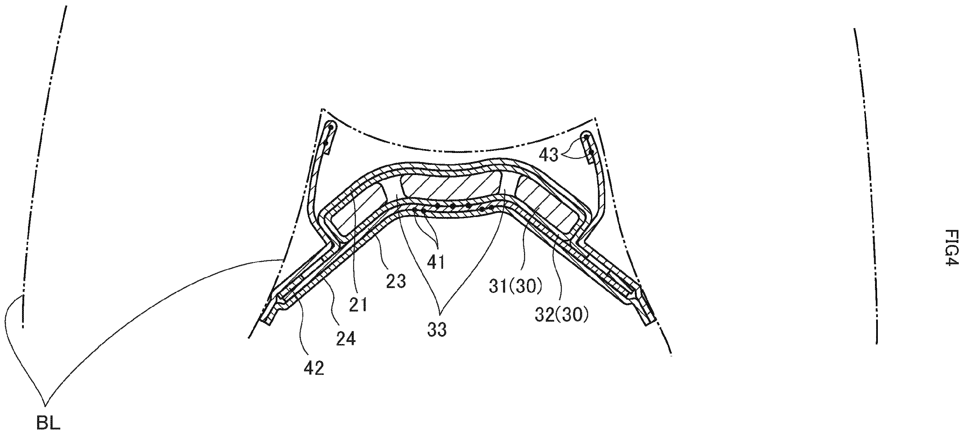

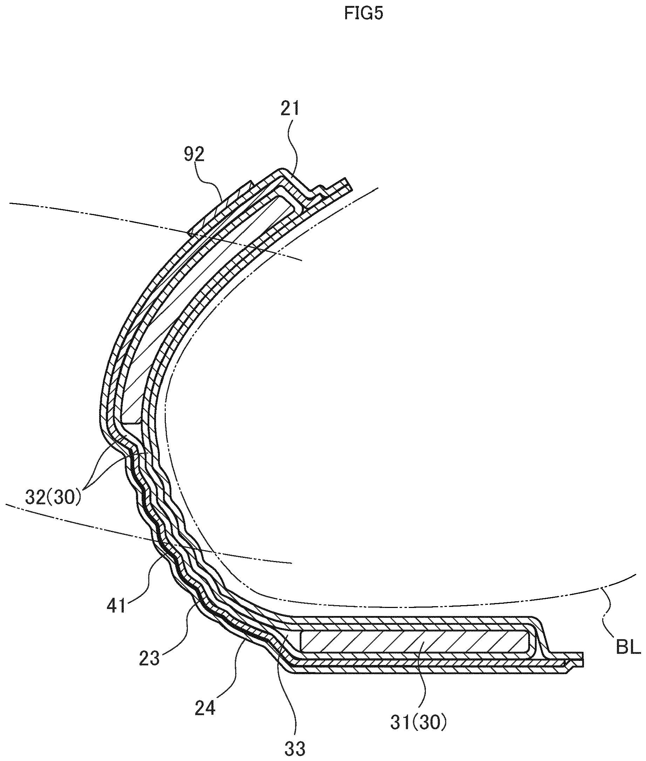

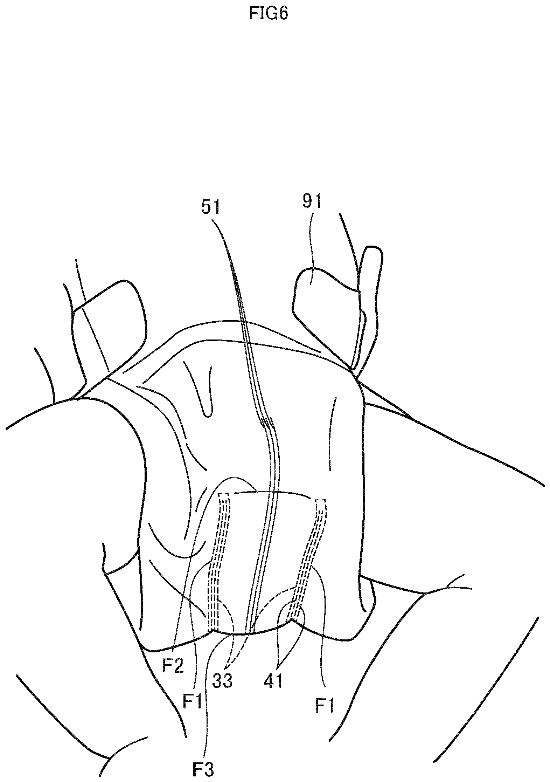

Next, the wearing state of the absorbent article 10 is described by referring to Figs. 4 to 6. Fig. 4 schematically illustrates a wearing state of the absorbent article in a cross-section taken along line A-A. Fig. 5 schematically illustrates a wearing state of the absorbent article in a cross-section taken along line B-B. Fig. 6 schematically illustrates a wearing state of the absorbent article according to the embodiment, in which a wearer is viewed from the front side while the wearer is lying. The line BL in Figs. 4 and 5 represents the body line of the wearer.

First, to put on the tape type absorbent article 10, the buttocks of the wearer are placed on the rear waistline region S2 of the absorbent article 10 with the absorbent article 10 deployed. Then, the absorbent article 10 is passed between the legs of the wearer, and the absorbent article 10 is pulled up so that the abdomen of the wearer can be covered by the front waistline region S1. When the absorbent article 10 is pulled up toward the wearer, the crotch region R13 is disposed to face the crotch of the wearer.

When the absorbent article 10 is pulled up to bring the crotch region R13 closer to the wearer at the time of wearing, the absorbent core 31 of the crotch region R13 deforms along the low basis weight portion 33 functioning as the base point, as the rigidity of the low basis weight portion 33 is lower than the rigidity of the surroundings thereof. The low basis weight portion 33 extends in the front-back direction L, and the base point of deformation is formed extending in the front-back direction L between the central region R13C and the side regions R13S. Since the contraction force of the central region R13C in the front-back direction L is higher than the contraction force of the side regions R13S, the central region R13C contracts in the front-back direction L than the side regions R13S and is pulled up toward the body. Therefore, as illustrated in Fig. 4, the absorbent core 31 in the crotch region R13 is deformed in a convex shape in which the central region R13C protrudes toward the body from the side regions R13S.

As the absorbent core 31 in the crotch region R13 deforms in a convex shape, the length of the entire absorbent core 31 in the width direction W is shortened at the time of wearing. The side flaps 15 hardly hit the legs of the wearer at the time of wearing, and the absorbent article 10 is easily pulled up. Since the absorbent core 31 in the crotch region R13 deforms in the convex shape, the ease of pulling up at the time of wearing is achieved. Accordingly, the length in the width direction W of the entire crotch region R13 is reliably provided and the absorption area of the body fluid is reliably provided.

Since the low basis weight portions 33 are separated in the width direction W, the region between the low basis weight portions 33 is disposed to face the excretion opening of the wearer. By disposing the region facing the excretion opening, the absorbing ability for the body fluid is improved and leakage is decreased. More preferably, as illustrated in Fig. 6, when the absorbent article 10 in the wearing state is viewed from the non-skin contact surface side T2, the absorbent article 10 may include a base point of deformation F1 along the outer edge of the low basis weight portions 33, a base point of deformation F2 along a line connecting the front edges 33F of the pair of low basis weight portions 33, and a base point of deformation F3 along a line connecting the rear edges 33R of the pair of low basis weight portions 33. A substantially rectangular region surrounded by the base points of deformation F1 to F3 is disposed to face the excretion opening of the wearer. Since the region surrounded by the base points of deformation F1 to F3 are disposed to face the excretion opening, the absorbing ability for the body fluid is improved and the leakage can be decreased.

As illustrated in Figs. 2 and 4, the central elastic member 41 is disposed in a region overlapping the central region R13C in plan view. By the contraction of the central elastic member 41, the absorbent core 31 in the crotch region R13 deforms in a convex shape in which the central region R13C protrudes toward the wearer from the side regions R13S. In addition, the central elastic member 41 may be disposed outside the center R13WL between the center R13CL of the central region R13C and the outer edge R13CE of the central region R13C in the width direction W. On the outer edge R13CE of the central region R13C, the low basis weight portions 33 functioning as the base points of deformation are provided. The central elastic member 41 is located outside the center R13WL of the center of the central region R13C and the outer edge of the central region R13C in the width direction W. This facilitates the contraction force by the central elastic member 41 to act on the low basis weight portions 33. The low basis weight portions 33 are easily pulled up toward the body by both the difference in rigidity due to the difference in basis weight and the contraction force of the central elastic member 41. Therefore, the absorbent core in the crotch region R13 becomes easily deformed in the convex shape.

The central elastic member 41 may be disposed so as to overlap the low basis weight portion 33 in plan view. According to such a configuration, the contraction force by the central elastic member 41 easily acts by the low basis weight portions 33. The low basis weight portions 33 are easily pulled up toward the body by both the difference in rigidity due to the difference in basis weight and the contraction force of the central elastic member 41, so that the absorbent core 31 can easily deform in the concave shape.

The central elastic member 41 may be disposed in a pair at a position apart in the width direction not overlapping the center in the width direction of the central region R13C in plan view. In the central elastic member of the present embodiment, two thread rubbers are each disposed separated from each other on the left and right sides. A region not overlapping the central elastic member 41 is provided between the low basis weight portions 33. A region not overlapping the central elastic member 41 is more likely to be disposed away from the body during wearing than a region where the central elastic member 41 is disposed and a region where the low basis weight portion 33 is provided. With the space formed between the low basis weight portions 33 and the body, it is possible to provide a space for reliably keeping the body fluid and decrease the leakage of the body fluid. Further, as illustrated in Fig. 6, the central elastic member 41 may be disposed outside the indicators 51 in the width direction W. Since the central elastic member 41 does not overlap the indicators 51, it is possible to prevent the decrease of contraction of the indicators 51 due to the contraction of the central elastic member 41. Accordingly, the visibility of the indicators 51, when visually recognized from the outside of the absorbent article 10, can be maintained.

More preferably, the central elastic member 41 may be disposed in a region that is separated in the width direction W and overlaps the low basis weight portion 33. According to this configuration, the region not overlapping the central elastic member 41 is more likely to be disposed away from the body when wearing, compared to the region provided with the low basis weight portion 33, and the low basis weight portion 33 overlapping the central elastic member 41 is easily placed near the body at the time of wearing.

In another embodiment, the central elastic member 41 may be formed by a stretchable sheet having a certain length in the width direction W. The stretchable sheet may be disposed across the pair of low basis weight portions 33.

Further, the central elastic member 41 may be disposed on the non-skin contact surface side T2 of the absorbent core 31. Since the central elastic member 41 is disposed on the non-skin contact surface side T2 of the absorbent core 31, the absorbent core 31 is easily pushed up toward the wearer by the central elastic member 41. The absorbent core 31 is brought close to the body by the central elastic member 41, and the absorbent core 31 is easily deformed in a convex shape more easily.

The central elastic member 41 may be disposed in a region not overlapping with the side regions R13S in plan view. That is, the central elastic member 41 may be disposed only in a region overlapping the central region R13C. Since the central elastic member 41 is not disposed in the side regions R13S, the central region R13C is more easily pulled toward the body than the side regions R13S. The central region R13C easily deforms in the convex shape protruding toward the body side. An effect of easily pulling up at the time of wearing is easily obtained, while reliably providing the absorption area of body fluid.

The central elastic member 41 may be disposed across the folding line FL formed by bending the absorbent article 10 in two in the front-back direction L. The folding line FL of the absorbent article 10 passes through the center in the front-back direction L of the absorbent article 10 and extends in the width direction W. The folding line FL of the absorbent article 10 may be disposed apart from the body at the time of wearing due to crease. Since the central elastic member 41 is disposed across the folding line FL, the crease can be brought closer to the body.

As illustrated in Fig. 1, the center 41CL of the central elastic member 41 in the front-back direction L may be located behind the center of the absorbent article 10 in the front-back direction L. By disposing the central elastic member 41 toward the rear of the absorbent article 10, the portion behind the center of the absorbent article 10 in the front-back direction L can be further contracted. On the portion behind the center of the absorbent article 10 in the front-back direction L, a region for placing the body (buttocks) at the time of wearing is provided. Since the region on which the body is placed and the region contracted by the central elastic member 41 are close to each other, it is easy to make the region extending from the body to fit the body at the time of wearing. Therefore, the region on which the body is placed during wearing is brought into close contact with the body by body pressure, and the region extending from the body is more likely to be brought into close contact with the body by the central elastic member 41. Further, the absorbent core 31 is easily disposed to fit the body, so that the absorbent core 31 is prevented from being caught by the legs at the time of wearing and is easily pulled up. Further, by disposing the absorbent core 31 to fit the body, it is possible to reliably provide the absorption area of the body fluid.

As illustrated in Fig. 1, the rear edge 41R of the central elastic member 41 may be located in front of the region S4 which corresponds to 1/3 of the entire length of the absorbent article 10 from the rear edge of the absorbent article 10 in the front-back direction L of the absorbent article 10. A region from the rear end edge of the absorbent article 10 to 1/3 of the entire length in the front-back direction L of the absorbent article 10 is likely to be a region on which the body of the wearer (buttocks) is placed at the time of wearing. Since the central elastic member 41 is not provided in the area where the body is placed at the time of wearing, the body is placed on the absorbent core 31 in a smooth state, and it is easy to dispose the absorbent core 31 to fit the body. Further, the absorbent core 31 is easily disposed to fit the body, so that the absorbent core 31 is prevented from being caught by the legs at the time of wearing and is easily pulled up. Further, by disposing the absorbent core 31 to fit the body, it is possible to reliably provide the absorption area of the body fluid.

The rear edge 41R of the central elastic member 41 may be located on the front side of the rear edge 33R of the low basis weight portion 33. In the area behind the low basis weight portion 33, the contraction force by the central elastic member 41 hardly acts, so that it is easy to maintain the smooth state. In the area behind the low basis weight portion 33, the rear end portion of the absorbent article 10 on which the body is placed at the time of wearing is easy to fit the body in the smooth state. Further, the absorbent core 31 is easily disposed to fit the body, so that the absorbent core 31 is prevented from being caught by the legs at the time of wearing and is easily pulled up. Further, by disposing the absorbent core 31 to fit the body, it is possible to reliably provide the absorption area of the body fluid. Further, the front edge 41F of the central elastic member 41 may be located behind the front edge 33F of the low basis weight portions 33. Since the central elastic member 41 is located inside the low basis weight portions 33 in the front-back direction L, only the region where the low basis weight portion 33 is disposed in the front-back direction L is contracted by the central elastic member 41 and low It is possible to dispose only the area where the low basis weight portion 33 is disposed closer to the body. It becomes easier to achieve a convex shape having the area where the low basis weight portion 33 is disposed as the vertex.

As illustrated in Fig. 1, a distance G33 between the low basis weight portions 33 may be not more than 1/2 of the maximum length 31M1 in the width direction of the absorbent core 31. The distance G33 between the low basis weight portions 33 is the distance in the width direction between the inner edges of the low basis weight portions 33. In the configuration in which the distance between the low basis weight portions 33 changes, the distance G33 in the longest length in the width direction. The portion between the low basis weight portions 33 is disposed so as to approach the body at the time of wearing. Since the distance G33 between the low basis weight portions 33 is not more than 1/2 of the maximum length 31M1 of the absorbent core 31, the low basis weight portions 33 are disposed between the legs of the wearer and the convex shape of the absorbent core 31 is easily maintained. The distance G33 between the low basis weight portions 33 may be not more than 1/2 of the minimum length 31M2 of the absorbent core 31 in the width direction W in the crotch region R13. Since the distance between the low basis weight portions 33 is not more than 1/2 of the minimum length of the absorbent core 31 in the crotch region R13, it is easy to dispose the low basis weight portions 33 between the legs of the wearer and maintains the convex shape of the absorbent core. Accordingly, the side flaps 15 hardly hit the legs of the wearer at the time of wearing, and the absorbent article 10 is more easily pulled up. The distance G33 between the low basis weight portions 33 may be from 30 mm to 35 mm. When the distance G33 between the low basis weight portions 33 is from 30 mm to 35 mm, it is easy to dispose the low basis weight portions 33 between the legs of the wearer and maintain the convex shape of the absorbent core 31.

The bending stiffness of the center region R13C in the width direction W may be higher than the bending stiffness of the side regions R13S in the width direction W. When the bending stiffness of the center region R13C in the width direction W is higher than the bending stiffness of the side regions R13S in the width direction W, the central region R13C can be easily maintained in a smooth state even when the central region R13C has the high contraction force. Further, the absorbent core 31 is easily disposed to fit the body, so that the absorbent core 31 is prevented from being caught by the legs at the time of wearing and is easily pulled up. Further, by disposing the absorbent core 31 to fit the body, it is possible to reliably provide the absorption area of the body fluid. The bending stiffness of at least a partial region of the central region R13C needs to be higher than the bending stiffness of the side regions R13S in the width direction W, so that the central region R13C may be formed in the same bending stiffness region as the side regions R13S.

In the center region R13C, at least the compressed portion 35 formed by pressing the absorbent core 31 may be formed. The bending stiffness of the central region R13C increases by the compressed portion 35, the central region R13C is maintained in a smooth state, and is easily disposed along the body. Further, the absorbent core 31 is easily disposed to fit the body, so that the absorbent core 31 is prevented from being caught by the legs at the time of wearing and is easily pulled up. Further, by disposing the absorbent core 31 to fit the body, it is possible to reliably provide the absorption area of the body fluid.

Further, the central elastic member 41 may be disposed outside the compressed portion 35 in the width direction, and may be disposed so as not to overlap the compressed portion 35 in plan view. The region where the central elastic member 41 is disposed is pulled up toward the body by contraction of the central elastic member 41, so that the region between the central elastic members 41 is easily disposed to fit the body by the compressed portion 35. The space between the central elastic members 41 is easily deformed in a convex shape to fit the body. Therefore, it is easy to pull up at the time of wearing while ensuring the absorption area of the body fluid.

In another embodiment, the bending stiffness of the central region R13C may be higher than the bending stiffness of the side regions R13S by any constituent component other than the compressed portion 35. Specifically, the bending stiffness of the central region R13C may be higher than the bending stiffness of the side regions R13S, because the basis weight of the bending absorbent core 31 is higher than the basis weight of the absorbent core 31 of the side regions R13S. Alternatively, by making the material overlapping the central region R13C larger than the material overlapping the side regions R13S, or by making the stiffness of the material overlapping the central region R13C higher than the rigidity of the material overlapping the side regions R13S, The flexural rigidity of R13C may be higher than the bending stiffness of the side regions R13S.

Further, in another embodiment, the compressed portion 35 may be disposed between the low basis weight portions 33. Since the compressed portion 35 is disposed between the low basis weight portions 33 in the width direction W, the region between the low basis weight portions 33 is maintained in a smooth state and is disposed along the body. Therefore, it is possible to reliably provide the absorption area of body fluid.

As used herein, the "basis weight of the absorbent core" is measured as described below. A sample of the absorbent article 10 is taken out from a package or the like, if packaged in a sealing manner, and left as it is for 12 hours under an atmosphere of 20°C ± 2°C and relative humidity of 60% ± 5% RH. For an absorbent article wrapped by a packaging sheet or the like, the packaging sheet is opened and the folded absorbent article is deployed. Subsequently, the part for measuring the basis weight is cut out from the absorbent article, and the part other than the absorbent core such as the topsheet is removed from the cut part. Then, the area and the weight of the part for measuring the basis weight of the absorbent core are measured. The basis weight is calculated in accordance with the weight and the area.

As used herein, the "flexural rigidity" in the present specification may be a value measured by Gurley bending softness. Measurement of Gurley bending resistance can be measured according to JIS-L1096 using a Gurley type flexibility tester of No. 311 manufactured by Yasuda Seiki Seisakusho, Ltd. The length in the width direction of the sample for measurement of the Gurley bending softness can be 30 mm and the length in the front-back direction can be 25 mm.

The embodiments of the present invention have been described above in detail, it is apparent for persons who have ordinary skill in the art that the embodiments described in this specification do not limit the scope of the present invention. Changes and modifications may apply to the embodiments of the present invention without departing from the scope of the present invention that are defined by the description of the appended claims.

### [INDUSTRIAL APPLICABILITY]

According to the present invention, it is possible to provide an absorbent article that is easily pulled up at the time of wearing and can reliably provide an absorption area of a body fluid.

### [REFERENCE SIGNS LIST]

- 10: Absorbent article
- 15: Side flap
- 21: Top sheet
- 22: Side sheet
- 23: Backsheet
- 24: Exterior sheet
- 30: Absorber
- 31: Absorbent core
- R11: Front region
- R12: Rear region
- R13: Crotch region
- R13C: Center region
- R13S: Side region
- 32: Core wrap
- 33: Low basis weight portion
- 41: Central elastic member
- 42: Leg elastic member
- 43: Leakage preventing elastic member
- 51: Indicator
- 91: Fastening tape
- 92: Target portion
- L: Front-back direction
- W: Width direction
- T: Thickness direction
- T1: Skin contact surface side
- T2: Non-skin contact surface side
- S1: Front waistline region
- S2: Rear waistline region
- S3: Intermediate region

## Claims

1. An absorbent article (10), comprising:
a front-back direction (L);
a width direction (W) orthogonal to the front-back direction;
an absorbent core (31) including an absorbent material;
a side flap (15) extending outward in the width direction from the absorbent core (31); and
a fastening tape (90) provided on the side flap (15), wherein
the absorbent article (10) includes a front region(R11), a crotch region(R13), and a rear region (R12) which are obtained by equally dividing a total length of the absorbent core (31) in the front-back direction into three parts,
a length of the side flap (15) in the width direction in the crotch region (R13) is at least 30 mm,
the crotch region includes a central region (R13C) and side regions (R13S) located on both outer sides of the central region in the width direction,
low basis weight portions (33) each having a basis weight of the absorbent material lower than a basis weight of the surroundings thereof are provided on both outer edges of the central region (R13C) in the front-back direction, and
a contraction force of the central region (R13C) in the front-back direction is higher than a contraction force of the side regions (R13S) in the front-back direction,
wherein the central region (R13C) includes a central elastic member (41) that stretches in the front-back direction.

2. The absorbent article (10) according to claim 1, wherein the central elastic member (41) is disposed outside the center (R13WL) of a region between the center of the central region (R13C) and the outer edges (R13CE) of the central region in the width direction.

3. The absorbent article (10) according to claim 1 or 2, wherein the central elastic member (41) is disposed to overlap the low basis weight portions (33).

4. The absorbent article (10) according to any one of claims 1 to 3, wherein the central elastic member (41) is disposed in a pair at a position not overlapping the center (R13CL) of the central region (R13C) in the width direction and separated from each other in the width direction.

5. The absorbent article (10) according to any one of claims 1 to 4, wherein the central elastic member (41) is disposed on the non-skin contact surface side (T2) of the absorbent core (31).

6. The absorbent article (10) according to any one of claims 1 to 5, wherein the central elastic member (41) is disposed in a region not overlapping the absorbent core (31) in the side regions (R13S).

7. The absorbent article (10) according to any one of claims 1 to 6, wherein
the central elastic member (41) is disposed across a folding line (FL) formed by bending the absorbent article in two in the front-back direction, and
the center (41CL) of the central elastic member (41) in the front-back direction is located behind the center of the absorbent article in the front-back direction.

8. The absorbent article (10) according to any one of claims 1 to 7, wherein a rear edge (41R) of the central elastic member (41) is located in front of a region equivalent to 1/3 of the entire length of the absorbent article in the front-back direction from the rear edge of the absorbent article.

9. The absorbent article (10) according to any one of claims 1 to 8, wherein the rear end edge of the central elastic member (41) is located in front of rear edges (33R) of the low basis weight portions (33).

10. The absorbent article (10) according to any one of claims 1 to 9, wherein a distance (G33) between the low basis weight portions (35) is not more than 1/2 of a maximum length (31M1) in the width direction of the absorbent core (31).

11. The absorbent article (10) according to any one of claims 1 to 9, wherein a distance (G33) between the low basis weight portions (33) is not more than 1/2 of the minimum length (31M2) in the width direction of the absorbent core (31) in the crotch region (R13).

12. The absorbent article (10) according to any one of claims 1 to 11, wherein a bending stiffness of the central region (R13C) in the width direction is higher than a bending stiffness of the side regions (R13S) in the width direction.

13. The absorbent article (10) according to claim 12, wherein the central region (R13C) includes a compressed portion (35) formed at least by pressing the absorbent core.

14. The absorbent article (10) according to claim 13, wherein the compressed portion (35) is disposed between the low basis weight portions (33).

15. The absorbent article (10) according to claim 13 or 14, wherein
the central region (R13C) includes the central elastic member (41) that stretches in the front-back direction, and
the central elastic member is disposed outside the compressed portion (35) in the width direction.

## Patentansprüche

1. Absorbierender Artikel (10), der Folgendes umfasst:
eine Vorn-Hinten-Richtung (L);
eine Breitenrichtung (W) rechtwinklig zu der Vorn-Hinten-Richtung;
einen Saugkern (31), der ein absorbierendes Material einschließt;
eine Seitenklappe (15), die sich nach außen in der Breitenrichtung von dem Saugkern (31) erstreckt; und
einen Befestigungsstreifen (90), der auf der Seitenklappe (15) bereitgestellt ist, wobei der absorbierende Artikel (10) einen vorderen Bereich (R11), einen Schrittbereich (R13) und einen hinteren Bereich (R12) einschließt, die durch gleichmäßiges Teilen einer Gesamtlänge des Saugkerns (31) in der Vorn-Hinten-Richtung in drei Teile erhalten werden,
eine Länge der Seitenklappe (15) in der Breitenrichtung in dem Schrittbereich (R13) mindestens 30 mm beträgt,
der Schrittbereich einen mittleren Bereich (R13C) und Seitenbereiche (R13S), die sich an beiden äußeren Seiten des mittleren Bereichs in der Breitenrichtung befinden, einschließt,
Abschnitte mit geringem Flächengewicht (33), die jeweils ein Flächengewicht des absorbierenden Materials aufweisen, das geringer ist als ein Flächengewicht der Umgebungen davon, an beiden äußeren Rändern des mittleren Bereichs (R13C) in der Vorn-Hinten-Richtung bereitgestellt sind und
eine zusammenziehende Kraft des mittleren Bereichs (R13C) in der Vorn-Hinten-Richtung größer ist als eine zusammenziehende Kraft der Seitenbereiche (R13S) in der Vorn-Hinten-Richtung,
wobei der mittlere Bereich (R13C) ein mittleres elastisches Element (41) einschließt, das sich in der Vorn-Hinten-Richtung dehnt.

2. Absorbierender Artikel (10) nach Anspruch 1, wobei das mittlere elastische Element (41) außerhalb der Mitte (R13WL) eines Bereichs zwischen der Mitte des mittleren Bereichs (R13C) und den äußeren Rändern (R13CE) des mittleren Bereichs in der Breitenrichtung angeordnet ist.

3. Absorbierender Artikel (10) nach Anspruch 1 oder 2, wobei das mittlere elastische Element (41) zur Überlappung mit den Abschnitten mit geringem Flächengewicht (33) angeordnet ist.

4. Absorbierender Artikel (10) nach einem der Ansprüche 1 bis 3, wobei das mittlere elastische Element (41) als ein Paar an einer Position angeordnet ist, das die Mitte (R13CL) des mittleren Bereichs (R13C) in der Breitenrichtung nicht überlappt und voneinander in der Breitenrichtung getrennt ist.

5. Absorbierender Artikel (10) nach einem der Ansprüche 1 bis 4, wobei das mittlere elastische Element (41) auf einer Nicht-Hautkontaktoberflächenseite (T2) des Saugkerns (31) angeordnet ist.

6. Absorbierender Artikel (10) nach einem der Ansprüche 1 bis 5, wobei das mittlere elastische Element (41) in einem Bereich angeordnet ist, der nicht mit dem Saugkern (31) in den Seitenbereichen (R13S) überlappt.

7. Absorbierender Artikel (10) nach einem der Ansprüche 1 bis 6, wobei
das mittlere elastische Element (41) quer über einer Faltlinie (FL) angeordnet ist, die durch Knicken des absorbierenden Artikels in der Vorn-Hinten-Richtung ausgebildet wird, und
sich die Mitte (41CL) des mittleren elastischen Elements (41) in der Vorn-Hinten-Richtung hinter der Mitte des absorbierenden Artikels in der Vorn-Hinten-Richtung befindet.

8. Absorbierender Artikel (10) nach einem der Ansprüche 1 bis 7, wobei sich ein hinterer Rand (41R) des mittleren elastischen Elements (41) vor einem Bereich befindet, der 1/3 der gesamten Länge des absorbierenden Artikels in der Vorn-Hinten-Richtung von dem hinteren Rand des absorbierenden Artikels entspricht.

9. Absorbierender Artikel (10) nach einem der Ansprüche 1 bis 8, wobei sich der hintere Endrand des mittleren elastischen Elements (41) vor hinteren Rändern (33R) der Abschnitte mit geringem Flächengewicht (33) befindet.

10. Absorbierender Artikel (10) nach einem der Ansprüche 1 bis 9, wobei eine Entfernung (G33) zwischen den Abschnitten mit geringem Flächengewicht (35) nicht mehr als 1/2 einer maximalen Länge (31M1) in der Breitenrichtung des Saugkerns (31) beträgt.

11. Absorbierender Artikel (10) nach einem der Ansprüche 1 bis 9, wobei eine Entfernung (G33) zwischen den Abschnitten mit geringem Flächengewicht (33) nicht mehr als 1/2 einer minimalen Länge (31M2) in der Breitenrichtung des Saugkerns (31) in dem Schrittbereich (R13) beträgt.

12. Absorbierender Artikel (10) nach einem der Ansprüche 1 bis 11, wobei eine Biegesteifigkeit des mittleren Bereichs (R13C) in der Breitenrichtung höher ist als eine Biegesteifigkeit der Seitenbereiche (R13S) in der Breitenrichtung.

13. Absorbierender Artikel (10) nach Anspruch 12, wobei der mittlere Bereich (R13C) einen komprimierten Abschnitt (35) einschließt, der zumindest durch Pressen des Saugkerns ausgebildet ist.

14. Absorbierender Artikel (10) nach Anspruch 13, wobei der komprimierte Abschnitt (35) zwischen den Abschnitten mit geringem Flächengewicht (33) angeordnet ist.

15. Absorbierender Artikel (10) nach Anspruch 13 oder 14, wobei
der mittlere Bereich (R13C) das mittlere elastische Element (41) einschließt, das sich in der Vorn-Hinten-Richtung dehnt, und
das mittlere elastische Element außerhalb des komprimierten Abschnitts (35) in der Breitenrichtung angeordnet ist.

## Revendications

1. Article absorbant (10), comportant :
une direction allant d'avant en arrière (L) ;
une direction allant dans le sens de la largeur (W) orthogonale par rapport à la direction allant d'avant en arrière ;
une partie centrale absorbante (31) comprenant un matériau absorbant ;
un rabat latéral (15) s'étendant vers l'extérieur dans la direction allant dans le sens de la largeur depuis la partie centrale absorbante (31) ; et
un ruban d'attache (90) mis en œuvre sur le rabat latéral (15), dans lequel
l'article absorbant (10) comprend une région avant (R11), une région au niveau de l'entrejambe (R13) et une région arrière (R12) qui sont obtenues en divisant de manière égale une longueur totale de la partie centrale absorbante (31) dans la direction allant d'avant en arrière en trois parties ;
une longueur du rabat latéral (15) dans la direction allant dans le sens de la largeur dans la région au niveau de l'entrejambe (R13) mesure au moins 30 mm,
la région au niveau de l'entrejambe comprend une région centrale (R13C) et des régions latérales (R13S) situées des deux côtés extérieurs de la région centrale dans la direction allant dans le sens de la largeur,
des parties à faible masse surfacique (33) ayant chacune une masse surfacique du matériau absorbant inférieure à la masse surfacique des parties environnantes de celui-ci sont mises en œuvre sur les deux bords extérieurs de la région centrale (R13C) dans la direction allant d'avant en arrière, et
une force de contraction de la région centrale (R13C) dans la direction allant d'avant en arrière est supérieure à une force de contraction des régions latérales (R13S) dans la direction allant d'avant en arrière,
dans lequel la région centrale (R13C) comprend un élément élastique central (41) qui s'étire dans la direction allant d'avant en arrière.

2. Article absorbant (10) selon la revendication 1, dans lequel l'élément élastique central (41) est disposé à l'extérieur du centre (R13WL) d'une région entre le centre de la région centrale (R13C) et les bords extérieurs (R13CE) de la région centrale dans la direction allant dans le sens de la largeur.

3. Article absorbant (10) selon la revendication 1 ou la revendication 2, dans lequel l'élément élastique central (41) est disposé pour chevaucher les parties à faible masse surfacique (33).

4. Article absorbant (10) selon l'une quelconque des revendications 1 à 3, dans lequel l'élément élastique central (41) est disposé sous la forme d'une paire au niveau d'une position ne chevauchant pas le centre (R13CL) de la région centrale (R13C) dans la direction allant dans le sens de la largeur et de manière séparée l'un de l'autre dans la direction allant dans le sens de la largeur.

5. Article absorbant (10) selon l'une quelconque des revendications 1 à 4, dans lequel l'élément élastique central (41) est disposé sur un côté formant surface non en contact avec la peau (T2) de la partie centrale absorbante (31).

6. Article absorbant (10) selon l'une quelconque des revendications 1 à 5, dans lequel l'élément élastique central (41) est disposé dans une région ne chevauchant pas la partie centrale absorbante (31) dans les régions latérales (R13S).

7. Article absorbant (10) selon l'une quelconque des revendications 1 à 6, dans lequel
l'élément élastique central (41) est disposé en travers d'une ligne de pliage (FL) formée en pliant l'article absorbant en deux dans une direction allant d'avant en arrière, et
le centre (41CL) de l'élément élastique central (41) dans la direction allant d'avant en arrière est situé derrière le centre de l'article absorbant dans la direction allant d'avant en arrière.

8. Article absorbant (10) selon l'une quelconque des revendications 1 à 7, dans lequel un bord arrière (41R) de l'élément élastique central (41) est situé devant une région équivalente à un tiers (1/3) de la longueur totale de l'article absorbant dans la direction allant d'avant en arrière depuis le bord arrière de l'article absorbant.

9. Article absorbant (10) selon l'une quelconque des revendications 1 à 8, dans lequel le bord d'extrémité arrière de l'élément élastique central (41) est situé devant des bords arrière (33R) des parties à faible masse surfacique (33).

10. Article absorbant (10) selon l'une quelconque des revendications 1 à 9, dans lequel une distance (G33) entre les parties à faible masse surfacique (35) ne fait pas plus de la moitié (1/2) d'une longueur maximum (31M1) dans la direction allant dans le sens de la largeur de la partie centrale absorbante (31).

11. Article absorbant (10) selon l'une quelconque des revendications 1 à 9, dans lequel une distance (G33) entre les parties à faible masse surfacique (33) ne fait pas plus de la moitié (1/2) d'une longueur minimum (31M2) dans la direction allant dans le sens de la largeur de la partie centrale absorbante (31) dans la région au niveau de l'entrejambe (R13).

12. Article absorbant (10) selon l'une quelconque des revendications 1 à 11, dans lequel une rigidité en flexion de la région centrale (R13C) dans la direction allant dans le sens de la largeur est supérieure à une rigidité en flexion des régions latérales (R13S) dans la direction allant dans le sens de la largeur.

13. Article absorbant (10) selon la revendication 12, dans lequel la région centrale (R13C) comprend une partie comprimée (35) formée au moins en comprimant la partie centrale absorbante.

14. Article absorbant (10) selon la revendication 13, dans lequel la partie comprimée (35) est disposée entre les parties à faible masse surfacique (33).

15. Article absorbant (10) selon la revendication 13 ou la revendication 14, dans lequel
la région centrale (R13C) comprend l'élément élastique central (41) qui s'étire dans la direction allant d'avant en arrière, et
l'élément élastique central est disposé à l'extérieur de la partie comprimée (35) dans la direction allant dans le sens de la largeur.
